# EUROPEAN PATENT APPLICATION

(11) **EP 0 859 230 A1**
(43) Date of publication of application: **19.08.1998**
(21) Application number: 98200371.7
(22) Date of filing: 09.02.1998
(51) Int. Cl.: G01N 27/327, G01N 33/543, G01N 33/548, G01N 33/58

(54) **Detection of analytes using electrochemistry**

(30) Priority: 10.02.1997 EP 97200369
(71) Applicant: Cranfield University, Cranfield, Bedfordshire MK43 0AL (GB)
(72) Inventor: Setford, Steven John, Cranfield, Bedfordshire MK43 0AL (GB)
(74) Representative: Matulewicz, Emil Rudolf Antonius, Dr.

(57) **Abstract**

The invention described in this document relates to methods and apparatus used to perform diagnostic assays in which the means of detection is based on electrochemical methods.

The detection of specific analytes is facilitated by the use of labeled materials that are capable of generating electrical signals under a given set of assay conditions. The preferred labels are enzymatic in nature and operate by generating or consuming electrochemically active species in the assay environment.

The assay is performed in a suitable flow cell or flowing liquid system device, incorporating a solid phase material located such that it is in intimate contact with the liquid being passed through the flow cell or flowing liquid system and also in close proximity to a working electrode. This electrode is poised at an appropriate potential against a reference electrode and is able to detect electrochemically active substances in the surrounding liquid. Normally the flow cell or flowing liquid system will also house a reference electrode and, if required, a counter electrode.

In a typical embodiment, a sample suspected of containing the analyte to be detected is mixed with a molecular species having specific binding affinity for this analyte. This molecular species is conjugated to an enzyme label. The labeled specific binding molecule- analyte complex is then immobilized onto the solid phase material, located in the vicinity of the working electrode. The flow cell or flowing liquid system is then rinsed with a solution prior to the addition of substrate solution for the enzyme label. The enzyme label serves to convert the substrate to a product, the extent of the reaction being related to the amount of bound analyte present. The depletion/production of electro-active material is monitored at the working electrode.

The methods and devices described by this invention are particularly suited for samples containing species that cause interference in conventional assays. The flow cell or flowing liquid system design allows removal of such species prior to measurement.

## Description

### Introduction

The invention detailed in this document describes a novel assay technique and apparatus for determining the presence and/or concentration of analytes in complex sample matrices. The invention utilises molecules having specific binding affinities for other molecules (usually the analyte(s) to be detected). Such assays are generally termed diagnostic assays, although they are not necessarily concerned with diagnosing illnesses or other such physical conditions.

The diagnostic field is a relatively well developed area in which many different assay systems and test formats have been developed for a wide range of analytes including hormones, pathogens (including viral and bacterial sources) drugs and antibiotics. The molecules having specific binding affinities for the analytes to be detected include antibodies (monoclonal, polyclonal), antibody fragments, receptors, nucleic acids or ligands. Many different binding molecules specific for a whole range of analytes are now readily available or can be routinely produced. In the context of this patent, the term 'immuno-' refers to any of the specific binding components listed above unless otherwise stated.

These assays operate on the principle of quantifying the extent of specific binding of the test analyte to the binding component. Therefore, care must be taken to ensure that substances that can affect the signal to be measured and are present as a result of non-specific binding interactions are first removed. Most assay procedures therefore include one or more washing steps, intended to remove such substances.

Many sample liquids, such as whole blood, milk and saliva may contain substances that can cause interference to the assay signal. Consequently, many assays lack the required sensitivity, speed and ease-of-use for a particular application.

These are important criteria for the development and operation of a successful assay procedure and few test systems are currently commercially available that fulfill all these requirements in a satisfactory manner. Often assay sensitivities are insufficient (often analyte concentrations as little as microgrammes per litre or less must be detectable which is difficult to achieve in the presence of interferents) . In the case when assay sensitivities are sufficiently high, this is normally at the expense of a number of washing and/or separation steps, necessitating the assay to be performed by trained personnel. Moreover, the various steps necessary to reach the required sensitivity and/or specificity result in prolonged assay times, unacceptable for many test applications.

### Assay Formats Incorporating Specific Binding Components

A short summary of two of the most common specific binding assay formats is given below.

### Sandwich type assay

A 'capture' molecule with specific binding properties (e.g. an antibody or receptor molecule) for a given analyte is immobilised onto a solid phase support (e.g. nitrocellulose membrane, plastic microtiter well or carrier beads). The immobilised molecule is then exposed to a sample suspected of containing the analyte of interest. Under appropriate conditions, the analyte will bind and hence be immobilised by the capture molecule. Separation of bound and unbound analyte is achieved with a washing step. A second binding component, labeled with a tracer molecule (e.g. enzyme, radio label, fluorescent label or colloidal sol particle) with specific affinity for the analyte is allowed to bind to the analyte-capture molecule complex. A further washing step is followed by the addition of enzyme specific substrate solution. The magnitude of the generated signal is directly proportional to the amount of analyte present.

### Competitive or non-competitive inhibition assay

A molecule with specific binding properties (binding partner) for a given analyte is immobilised onto a solid phase support as described for the sandwich type assay. Analyte present in a sample competes for these specific binding sites with an added analyte analogue, conjugated to a label (e.g. enzyme label, radio label, fluorescent label or colloidal sol particle). After a washing step, the label is detected according to appropriate techniques. The magnitude of the signal generated is inversely proportional to the amount of analyte present in the sample.

### Common Detection Methods

The presence of a label can be determined by several methods. For example, the presence of an enzyme label can be determined by chromogenic, fluorescent, luminescent or electrochemical means.

Chromogenic based assays generally involve simple laboratory procedures but suffer from long incubation times and relatively poor levels of sensitivity. Nevertheless, this approach is routinely used. One approach of particular interest and wide-spread use are the so-called 'dipstick' tests. These test devices incorporate, within their design, all of the assay components necessary for analyte detection coupled to a wicking device and is a one-step process. The simplicity of device operation and low costs involved have resulted in a large market for this type of product. Assays of this type have been described in the literature.

Electrochemical assays focus on monitoring electrochemically active substances, either generated or consumed by a redox or other enzyme located at the surface of a suitable electrode. A very well known example of this type of assay is the use of the enzyme glucose oxidase, immobilised at an electrode surface, for the determination of glucose in the blood of diabetics. Similarly, cholesterol oxidase can be employed to quantify cholesterol levels in blood or serum. Many types of such enzyme electrodes' have been described in the literature.

Ideally, the immobilised enzyme component will be of the redox type. In this way, the electrochemically active species generated or consumed by action of the enzyme can be directly monitored at a suitable electrode surface. Unfortunately, there are many substances of clinical or industrial importance that do not have a suitable redox enzyme counterpart.

Non-redox enzymes can be employed in enzyme electrodes, such as the use of penicillinase for the detection of penicillin. The secondary reaction product of the penicillinase reaction are hydrogen ions which can be detected electrochemically using a standard pH probe. Such systems tend to have very poor sensitivity and are prone to interference.

A potential advantage of electrochemical detection methods over other competing methodologies is the speed in which a measurable signal can be obtained. For example, a traditional ELISA test may require hours in order to develop a measurable signal, compared with minutes for an equivalent electrochemical process. This approach therefore has the obvious advantage of reducing total assay times.

### Electrochemical Immunoassay Systems

To circumvent these problems and increase the number of analytes that can be detected by electrochemical means, much recent work has focused on the development of electrochemical immunoassay systems. In this approach, a biocomponent that has a specific binding affinity for the analyte of interest, or an analyte analogue, is immobilised within the system (the binding component is usually an antibody or receptor molecule). Analyte detection requires the addition or removal of a tracer material, that has specific binding affinity for the biocomponent, analyte or analyte analogue and which is conjugated to a suitable enzyme label. The amount of immobilised enzyme present serves as a measure of the amount of analyte present in the sample.

Addition of saturating levels of enzyme substrate to the system will result in consumption of substrate at a rate dependent upon the amount of immobilised enzyme present. The depletion of substrate or generation of product can be monitored as appropriate. Typical enzyme labels include glucose oxidase (the reaction by-product, hydrogen peroxide can be detected electrochemically), alkaline phosphatase (the substrate 1-napthyl phosphate is converted to the electrochemically active product napthol) and horseradish peroxidase.

Electrochemical immunosensor systems described in the literature often lack sensitivity and suffer from poor reproducibility, although the latter factor can be enhanced by repeated use of the same sensor device (**Patent GB 2289339**). The separation of the working electrode from the counter and reference electrodes has been described in an attempt to increase reproducibility. However, such systems are not user-friendly as all 3 electrodes require separate assembly. Sensor devices designed for repeated use still require periodic replacement, mainly due to fouling effects caused by deposition of materials on the sensor surfaces that can affect device performance. System calibration is necessary each time a new sensor device is used. For these reasons, there are few, if any, examples of truly reliable electrochemical immunosensors currently commercially available.

Current state-of-the-art electrochemical immunosensors reported in the literature generally require total assay times of at least 30 minutes to allow for adequate incubation times, necessary for the specific binding reaction to occur. The use of liquid flow technology to shorten incubation times has been described in the literature. Most of these systems employ a flow channel to sequentially introduce a number of test reagents into the detection unit of a flow cell or flowing liquid system device.

Methods for the improvement of sensitivity and minimisation of assay time using permeable electrodes in flow cell or flowing liquid system devices have been described. However, these systems still do not operate over a useful analytical range of concentrations when assay times are reduced to 10 minutes or less and also suffer from poor reproducibility. Means of ensuring that the electrochemically active end-product has the opportunity to contact the working electrode device prior to being transported from the system in the flowing liquid stream, thus decreasing the sensitivity of the system have not been addressed. This approach also appears to be susceptible to electrode fouling effects.

EP-A2-0352138 and EP-A2-0525723 both describe the use of a device that incorporates membranes as solid phase supports in conjunction with a solid electrode assembly. These types of electrode assembly are intended as disposable units to be used with appropriate instrumentation.

The invention herein described represents an improvement over existing technology by providing a rapid, sensitive, reproducible method for the electrochemical detection of analytes, without the problems associated electrochemically active interferents that may be present in the sample.

### The Invention

The invention provides a method for determining the presence and/or the amount of at least one analyte in a liquid sample, comprising providing a molecule having specific binding affinity for said analyte, providing a label capable of generating or consuming an electrochemical signal detectable by an electrode, whereby the liquid sample flows along the electrode and whereby a solid phase is brought in close vicinity with said electrode, which solid phase is capable of binding a molecule having the label, whereby the presence of said label is detected by said electrode allowing determination of the presence and/or the quantity of analyte in the sample. The flow of any liquid may be halted at any time during the process.

By providing a flow cell or flowing liquid system in which the sample and following liquids can reproducibly contact an electrode and immobilised assay components, the problems associated with non-specific binding of interfering substances is significantly reduced, probably due to the flow of liquid through the system removing non-specifically bound substances. Specifically bound material should be substantially unaffected by said flow of liquid. Advantageously, the liquid sample is transported from one side of at least the said electrode to the other side thereof, in such a way that the liquid passes said electrode in an essentially non-turbulent manner, such that a substantial proportion of the liquid sample is able to come into contact with said electrode.

Preferably, the electrode devices are mass-fabricated using screen-printing methodology, a simple, cost-effective approach, or other suitable depositioning techniques. The solid phase material is in the form of a sheet of porous material, preferably nitrocellulose or is the electrode itself. The specific binding components of the assay (receptor proteins, antibodies, antibody fragments, strands of nucleic acid or other ligands) can be coated onto the solid phase support by simple protocols. Preferred labels according to this invention are enzymes which act to produce or consume electrochemically active substances to an extent that these reactions are measurable at an electrode surface. Such enzymes include oxidases, reductases, peroxidases and the like, which may originate or be derived from any microorganism or any other species, the only essential requirement being that a signal detectable by an electrode is produced upon generation or depletion of the electro-active material.

Depending on the analyte to be determined, all immunoassay formats (sandwich, competition, inhibition, agglutination) can be performed using the technology of this invention. The limitations that apply to all immunoassay procedures also apply to this process, such as the case where the detection of low molecular weight analytes is not readily achievable using the sandwich assay format.

Immunological interactions are a preferred embodiment of the present invention due to their wide applicability and specificity. It is well known in the art how to obtain polyclonal antisera, monoclonal antibodies and/or fragments and/or derivatives thereof, as well as how to produce genetically engineered antibodies or fragments or derivatives thereof. Another preferred interaction between analyte and specific binding molecule is the one whereby the analyte is a ligand for which the specific binding molecule is the receptor.

The invention described herein is particularly suited to determining the presence or concentration of analyte in complex matrices such as milk and whole blood, traditionally difficult materials in which to conduct assays. The assay incorporates the use of a flow cell or flowing liquid system device or equivalent apparatus allowing the ordered, sequential addition of assay reagents in a reproducible manner. A preferred way of carrying out the method of the invention comprises the use of the apparatus according to the invention. The invention provides an apparatus comprising a flow cell having a means, preferably a channel, through which liquid can flow whereby said means is provided with a working electrode, and at least one of a reference electrode and a counter electrode, a solid phase comprising a specific binding molecule capable of binding the molecule having the label, in close vicinity to said working electrode, said means having an inlet capable of being connected with one or more sources selected from the group consisting of a first source of a sample suspected of comprising an analyte, a second source of a liquid comprising a substrate, a third source of a liquid comprising a molecule having specific binding affinity for the analyte and a fourth source of a molecule having a label, whereby one or more of these sources may be one and the same, said means is provided with a liquid outlet, said electrodes suitable of being connected to a measuring device and the liquid in and/or outlet suitable of being connected to a liquid flow regulating means to flow the liquid.

A more detailed description of the embodiments of the invention is given below.

### Detailed description.

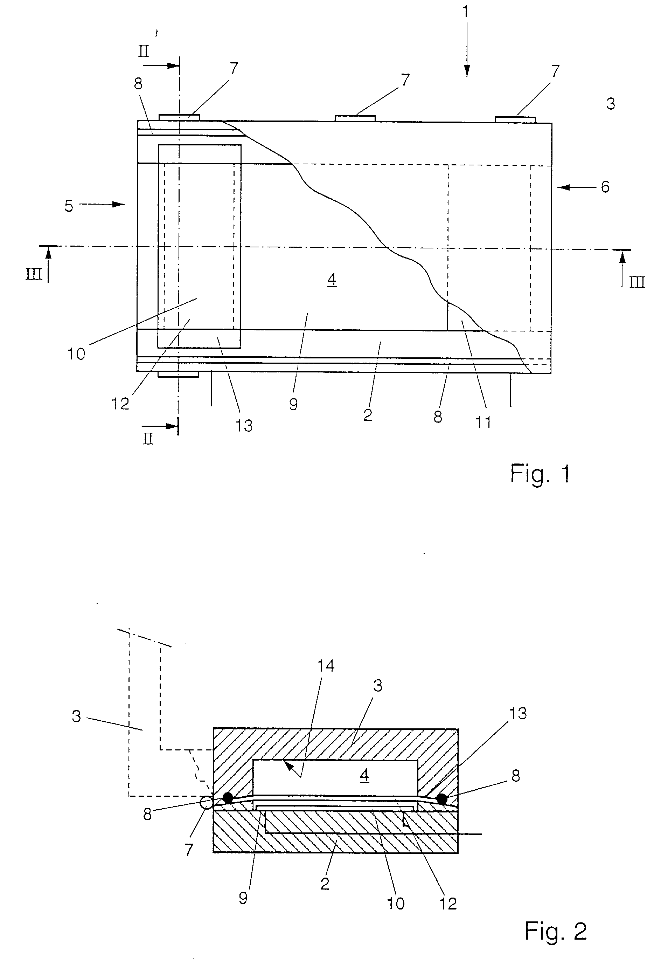

Figure 1, illustrates a cross-sectional view through a possible configuration of a flow cell or flowing liquid system immunoassay device which may be attached to a pump and measuring apparatus for test purposes.

Flowing stream systems have a number of potential advantages over the 'dipstick' type devices. In addition to the ease of use associated with flowing liquid methods, the passage of liquid sample and reagents over the transducing element is highly uniform and can be precisely controlled, thus contributing to enhanced device reproducibility. Furthermore by using a means preferably flow channels or similar vessels of appropriate dimensions, the interaction between the various reagent components (receptor protein/antibody and analyte/antigen) can be maximised when compared with the dip-stick type approach and standard static incubation methods (such as ELISA). By enhancing interactions between the various reagent species, a reduction in incubation time and hence a more rapid test system can be developed. Improvements in device sensitivity are also possible using this type of approach.

The basic operating principle of an amperometric biosensor device is the detection of an electro-active species, whose production or consumption can be related to the concentration of a particular analyte.

As an example, the case where glucose oxidase is used as an enzyme label in an electrochemical immunoassay system can be considered. In the presence of the enzyme substrate (glucose) under appropriate conditions, glucose oxidase enzyme will oxidise glucose and transfer electrons to dioxygen, forming the electrochemically active compound hydrogen peroxide. Hydrogen peroxide can be oxidised at a suitable electrode surface poised at an appropriate voltage against a standard reference electrode (typically +0.05 - 1.0 V, preferably +100 - 800mV, versus e.g. a silver/silver chloride reference). Electrons are transferred from the hydrogen peroxide to the electrode and associated circuitry, resulting in a current that can be measured amperometrically using suitable monitoring equipment. Other enzyme labels can be used that result in the production or consumption of an electro-active species and incorporating detection of said species at an electrode poised at an appropriate potential (positive, zero or negative) versus an appropriate standard electrode.

A major drawback of flowing liquid or flow injection systems, incorporating analyte detection using enzyme labels, is that the continuous flow of liquid serves to draw the material to be detected away from the transducer site. This will result in a decrease in system sensitivity. This is a particular problem when using flowing liquid or flow injection techniques in conjunction with electrochemical detection methods. This problem is reduced using the technology of this invention for the reasons described below.

### Advantages of the Invention

This invention describes the use of low-cost disposable thick-film screen-printed electrodes combined with flowing streams or flow injection analysis for the detection and quantification of trace analytes in process liquids. The current device and assay design has been shown to result in improvements of importance to at least 7 aspects of operation of such set-ups operated as electrochemical affinity sensors.
1) Due to the nature of the design of the assay procedure and device, the electrochemically active species to be detected (e.g. hydrogen peroxide) is effectively confined to a layer that is in close proximity to the surface of the transducing element. This factor acts to reduce losses in the electrochemically active species caused by flowing liquid stream transport effects. The material therefore has a greater opportunity to interact with the transducing element before being transported from the system.
2) The nature of the design of the assay procedure and device, ensures that, in addition to the electrochemically active species accumulating in the vicinity of the transducer, the enzyme label; the electrochemically active species is also located in the same vicinity.
3) Signal-noise ratios could be markedly reduced when operating the device as a 2 electrode, rather than a 3 electrode system. In a 2 electrode system, the reference electrode serves as both a reference and counter electrode, unlike a 3 electrode system, where working, reference and counter electrodes are provided. Further device improvements were possible by incorporating 2 working electrodes into the device design, whereby one of the working electrodes (primary electrode) is in close vicinity to the solid phase material, allowing quantification of both the Faradaic and non-Faradaic response of the system, whereas the other electrode serves as a compensator electrode, allowing the non-Faradaic background current to be subtracted from the primary electrode response.
4) The particular design of the device and assay format results in the generation of stable and reproducible signals, allowing signal measurements as initial velocities (measured as nA per second) to be recorded. This rapid measurement technique will allow a reduction in total assay time.
5) The combination of flowing stream or flow injection techniques with screen-printed thick-film sensor electrodes serves to minimise problems associated with system fouling, thus leading to an improvement in device reproducibility.
6) The design of the flow injection/flowing stream system and assay method is such that the obligatory washing steps, necessary in almost all immunological assay formats, can be performed using the substrate solution. The practical implications are that a time consuming extra step in the assay procedure can be omitted, thus further decreasing the total assay time. Electrochemical detection can commence after the substrate washing step. The design of the assay device is such that a minimal amount of substrate solution is required for the washing operation.
7) Screen-printing methodology represents a means of reproducibly mass-producing sensor devices. However, it is well known that variances between batches of screen-printed sensor devices can occur. For that reason, it has proved necessary to implement a standardisation procedure prior to assays being performed with each batch of cards.

The following scheme is proposed for the development of a commercial system. A number of sensor devices from each batch of cards can be tested as negative and positive controls at the production facility. By determining the signals from these cards at predetermined times, or by measuring the initial substrate conversion velocities obtained from a range of standard samples, the data generated can be employed to predict the behaviour of a particular batch of cards. The data can be programmed into a calibration card supplied with the sensor cards in a test kit. The card is programmed to set the measuring apparatus to a particular zero level. A simple software programme would allow the measuring device to consider samples yielding signals with a given degree of deviation from this zero setting to be either positive or negative with respect to the amount of analyte in the sample.

This is a simple process that has been used in commercially available enzyme electrode systems. The current invention relates to the case where pre-calibration of the system using enzyme-binding component sensor devices drawn from a particular batch is undertaken. This approach enables an end-user to perform a single test with a single sensing card, thus negating the need of calibrating the device every time a new test is performed.

### Flow cell or flowing liquid system design

The following equipment description serves as an example only. This invention covers all types and configurations of flow cell and flowing liquid systems that can be constructed and employed as per the other information provided in this specification.

The flow cell or flowing liquid system can be constructed such that a thick-film screen-printed electrode card can be mounted in place within the structure. The card is clamped or placed in the cell in such a way that sample liquid entering the system first encounters counter, working and then reference electrodes. A suitable seal or sealing method is used to prevent leakage of assay reagents during passage of the flowing streams. Figure 1. illustrates a schematic cross-section of a typical flow cell or flowing liquid system device.

The flow cell or flowing liquid system can be modular in design. The actual sensing area comprises a working electrode and an immobilisation support. Immunological reagents are immobilised onto this solid support. This support can be made of a suitably porous polymeric material, such as a flat-sheet nitrocellulose or nylon membrane and is ideally constructed such that the majority of the immunological reaction takes place on the side of the support that is positioned over and faces the transducing area.

A card so-prepared is mounted into the modular flow cell or flowing liquid system such that the electrodes are positioned in a hollow means e.g. channel or similar shaped vessel where fluid transport can occur, preferably in a predominantly non-turbulent manner, once the pumping or wicking of solution is started. Clamping of the card into the flow cell or flowing liquid system apparatus serves to maintain the solid phase support in it's position relative to the working electrode.

The flow cell or flowing liquid system device is further constructed in such a way that electrical contact can be made with electrically conducting contact pads that connect the electrodes to the measuring device.

A pump can be connected to either the inlet or the outlet of the flow cell or flowing liquid system device such that liquid is either pumped into or sucked out of the system in an ordered and reproducible manner. The system is then ready for testing.

A flow of sample is guided across the sensing card through the flow cell. Analytes present in the test sample will bind to the specifically impregnated immunological support. The flow cell or flowing liquid system is equally suited to perform both types of inhibition assay, sandwich assays and other types of assay.

The pumping of a sample liquid across the sensing card will result in a specific binding reaction between the immobilised binding component and any analyte present in the sample, provided that appropriate conditions are applied. After the passage of a fixed amount of sample, a valve is switched such that substrate solution of a appropriate concentration is passed through the system. The presence of immobilised tracer enzyme will result in the production or consumption of electrochemically active material. Given that the reaction site of the immunological support is faced towards the working electrode, the majority of electro-active material will be formed or consumed in between the support and working electrode surface, thus 'trapping' the product of the enzyme reaction in the vicinity of the working electrode. This serves to increase the time that this material is in close contact with the working electrode surface and therefore increases overall device sensitivity.

Before any considerable amount of electrochemically active material is formed or consumed, the substrate solution will act as a washing buffer, rinsing the immunological support and removing non-bound and interfering material from the flow cell or flowing liquid system device to a waste reservoir. Actual measurement is started an appropriate number of seconds after the substrate solution, acting as wash buffer, first reaches the support and electrodes. Total assay times of less than 5 minutes have been achieved using this type of approach.

Measurement at this point can be carried out in at least two ways. Either the flow of substrate into the flow cell or flowing liquid system is halted and the system is left for a fixed period of time to allow a certain amount of material to be formed or consumed, or the initial velocity of substrate conversion by the enzyme is determined. This latter approach enables one to perform a more rapid test since no waiting for accumulation of end-product is necessary. Both approaches indicate the amount of enzyme present on the solid-phase support and so are indicative (either directly or indirectly) of analyte concentrations in the test sample.

Since there can be variability between batches of screen-printed electrodes, a small number of cards can be taken from a production batch of cards and used to calibrate the system. In this way, the supply of just one calibration card with each batch of test cards is all that is necessary for calibrational purposes. The calibration card is programmed to set the measuring apparatus to a certain zero level. Software is then used to determine whether the initial signal velocity profile obtained for a particular test sample is higher or lower than the calibration card value. The device readout will record a positive or negative readout accordingly.

The flow cell or flowing liquid system device, sensor card and test procedure also allows quantitative data to be obtained. A co-supplied set of standard sensor cards enables an end-user to create a standard calibration curve. An unknown sample can then be run in the flow cell or flowing liquid system device and the signal generated can be compared to the standard curve and the concentration of specific analyte determined.

In the case where a solid phase (e.g. nitrocellulose membrane) is used, the fixing of the sensor card into the flow cell or flowing liquid system acts to retain this material at the working electrode surface.

Process fluids are introduced to the flow cell or flowing liquid system using a suitable pump (e.g. peristaltic pump) capable of delivering liquid flow rates, typically between 0.05 and 1.0 ml per minute.

Liquid flow devices have previously been described as methods of choice for improving assay sensitivities and reducing assay times. The use of enzyme electrodes for the electrochemical detection of specific analytes in process liquids is also very well known, the specificity of such devices being reliant on the nature of the enzyme system chosen. ,Electrodes prepared from a variety of suitable conducting materials and in a variety of sizes and configurations are used. Screen-printing as a means of manufacturing electrodes is of increasing interest given the relatively low costs involved and high volume throughputs that can be achieved. Data, obtained from approaches described in this patent, showed that a combination of electrochemistry, immunology, screen-printed electrode systems in flow cell or flowing liquid system formats yielded test systems of greater reproducibility and sensitivity than any other device formerly presented. An additional advantage is that the assay systems herein described required no toxic or otherwise unsafe substance.

### Electrode design

A 3 electrode design card was produced using standard screen-printing technology.

Both the working and counter electrodes were fabricated from carbon based ink paste. The reference electrode, a silver/silver chloride electrode was fabricated using a commercially available silver/silver chloride ink.

The electrodes were designed in such a way that, once mounted in the flow cell or flowing liquid system device, the liquid flows in an orderly manner across each of the electrodes in turn.

Basal tracks, used as a means of connecting each electrode to the potentiostat and monitoring device, were fabricated from a suitable conducting material, preferably ink. These tracks were designed in conjunction with the flow cell or flowing liquid system in order to facilitate simple connection to the potentiostat and monitoring device.

Once the system has been fully assembled, the whole immunological sequence of events will take place inside the flow cell or flowing liquid system at the surface or immediate vicinity of the working electrode.

The flow cell or flowing liquid system may incorporate connectors to allow simple attachment to a pumping device. The cell body can be made of any suitable inert material. Polymeric materials such as perspex appear particularly suited for this purpose. The flow cell or flowing liquid system can be made in two parts to allow introduction and removal of sensor cards into the flowing liquid streams.

### Example 1

Strips of nitrocellulose membrane were cut to the exact size of 0.4 x 0.9 cm. Strips were soaked in a solution of 5 mg/ml PBP (penicillin binding protein) in buffer (0.1M phosphate buffered saline (PBS), pH 7.4). The strips were left to soak for 1 hour at ambient temperature.

After incubation, strips were blocked in a 2% w/v bovine serum albumin (BSA) solution in PBS buffer (pH 7.4) for 1 hour at ambient temperature.

Individual strips were removed from the blocking solution and incubated for 5 minutes at 64°C in a 1:2000 dilution of 7-ACA-GOD stock solution in whole milk with or without free analyte (penicillin G) present.

Strips were washed for 20 seconds in PBS and placed onto the working electrode of the screen-printed sensor card.

The card was mounted into the flow cell or flowing liquid system and a 0.1M PBS solution was introduced, pumped at a constant rate through the flow cell. The working electrode was poised at a potential of +350 mV and the system allowed to achieve electrochemical equilibrium. A 0.1M glucose solution in 0.1M PBS/0.1M KCl was then pumped into the system. The flow was halted after 60 seconds. The electrochemical response of the system was monitored throughout this period.

Data obtained using this approach are presented in table 1. Results can be expressed as total current increase in µA over a given time interval or can be interpreted as current increase per unit time (µA/sec).

Measuring the initial response/time profile of the system is indicative of β-lactam concentration in the test sample and reduces the total assay time of the process.

**Table 1**

| Batch | Penicillin conc. (ppb) | Absolute response (µA) | | Velocity (nA sec¹) | |
|---|---|---|---|---|---|
| | | Mean (CV) | % of 0 ppb | Mean | % of 0 ppb |
| | | | | | |
| Overall | 0 (5) | 0.131 (27.3) | 100.00 | 0.411 (41.7) | 100.0 |
| | 10 (3) | 0.064 (17.7) | 49.00 | 0.151 (32.0) | 36.76 |
| | 100 (4) | 0.057 (70.2) | 43.26 | 0.135 (64.9) | 32.92 |
| | blank (6) | 0.003 (n/a) | 2.45 | 0.039 (n/a) | 9.42 |
| | | | | | |
| Batch 1 | 0 (3) | 0.141 (26.6) | 100.00 | 0.511 (23.4) | 100.00 |
| | 10 (0) | - (-) | - | - (-) | - |
| | 100 (2) | 0.087 (35.8) | 61.70 | 0.209 (12.6) | 40.90 |
| | blank (4) | 0.004 (n/a) | 2.48 | 0.159 (n/a) | 31.10 |
| | | | | | |
| Batch 2 | 0 (2) | 0.115 (32.7) | 100.00 | 0.261 (45.5) | 100.00 |
| | 10 (3) | 0.064 (17.7) | 55.90 | 0.151 (32.0) | 57.85 |
| | 100 (2) | 0.026 (21.8) | 22.71 | 0.062 (5.7) | 23.56 |
| | blank (2) | 0.003 (n/a) | 2.18 | 0.000 (n/a) | 0.00 |
| | | | | | |

Table 1: Data for nitro-cellulose incubation studies. Results are recorded as mean values of n repeat experiments (where n is listed in brackets). Velocity was measured over a time interval of 200 sec.; absolute response values were recorded over 500 sec.; results are also expressed as percentage of the signal of the positive control (0 ppb = no inhibition). CV values, in percent are recorded in brackets.

### Results: example 1:

Nitro-cellulose strips were prepared in two separate batches. The data are recorded as 'Overall' (batch 1 + batch 2) and batch 1 and batch 2 and shown in Table 1. Responses are recorded as 'absolute response' values, whereby the total current change is measured from the time that the glucose substrate reaches the WE to a time 500 sec. after this point. Responses are also recorded as 'velocities' whereby the maximum current versus time slope is measured during the period of glucose flow across the working electrode surface (expressed as nA/sec). Results are also expressed as a percentage of the signal of the zero ppb penicillin positive control, that is, the zero inhibited response that therefore yields a 100% response value.

### Example 2

Strips of nitrocellulose were cut to the exact size of 0.4 x 0.9 cm. Strips were soaked in a solution of 5 µg/ml of monoclonal anti-gentamycin antibody in PBS buffer pH 7.4, for 1 hour at ambient temperature.

After incubation, strips were blocked in a 2% w/v BSA solution in PBS(pH 7.4) for 1 hour at ambient temperature.

Individual strips were removed from the blocking solution and mounted onto the working electrode surface, thus creating a complete sensor card.

Sensor cards were mounted into the flow cell, the strip of nitrocellulose being held in place by a rubber seal, used to prevent leakage during operation of the flow cell device.

One ml samples of milk were aliquoted, to which were added 25 µl of a: 1:1000 (A), 1:2000 (B) or 1:2500 (C) dilution of gentamycin-GOD conjugate (prepared with state of the art procedures).

Free gentamycin was then added to each milk sample, yielding solutions having gentamycin concentrations varying between 0 and 1000 ppb.

A pumping device was connected to the flow cell and the prepared milk samples were each tested with a new sensor card mounted in place.

The flow rate through the system was adjusted so that the complete milk sample passed through the system over a 3 minute time period. A 1 minute wash step was then performed with 0.1 % w/v Tween in 0.1M PBS (pH 7.4). Immediately after each wash step, 0.5M glucose in 0.1M PBS/ 0.1M KCl was passed through the flow cell and the electrochemical responses generated were recorded both as absolute values and as initial response-time velocities in nA/sec.

A 1:10 dilution of the MoAb stock solution was used to coat the nitro-cellulose strips. Gentamycin-glucose oxidase conjugate concentrations were varied from 1:4000 (exp.A) to 1:8000 (exp.B) to 1:10 000 (exp. C). Results from these experiments are presented in Table 2.

**Table 2**

| Exp. No. | Gentamycin conc. | Absolute response (µA) | | Velocity (nA sec¹) | |
|---|---|---|---|---|---|
| | | Mean value | CV % | Mean value | CV % |
| | | | | | |
| A | 0 ppb | 3734 | 1.8 | 37.3 | 5.4 |
| | 10 ppb | 3217 | 3.6 | 36.0 | 0 |
| | 100 ppb | 3050 | 1.2 | 22.7 | 0 |
| | 1000 ppb | 1350 | 13.6 | 8.7 | 38.7 |
| | | | | | |
| B | 0 ppb | 2655 | 0.2 | 21.5 | 0 |
| | 10 ppb | 2438 | 1.5 | 18.0 | 2.8 |
| | 100 ppb | 2025 | 3.7 | 14.3 | 5.3 |
| | | | | | |
| C | 0 ppb | 2278 | 4.1 | 14.4 | 11.9 |
| | 10 ppb | 1868 | 2.6 | 10.8 | 10.8 |
| | 100 ppb | 1333 | 3.8 | 6.0 | 3.3 |
| | | | | | |

Table 2: Data from experiments a, b, c. Results are recorded as mean values of duplicate experiments (a, b) and triplicate experiments (c).

### Example 3

The purpose of this example is to demonstrate the applicability of the invention to performing highly sensitive assays using the so-called 'endpoint' measurement technique to quantify the level of enzyme activity, hence determining free gentamycin concentration in gentamycin containing milk samples.

The immunological reaction step is performed in a microtiter plate. Using GOD as the enzyme label, the final microtiter well solution will contain hydrogen peroxide, the amount of which is inversely proportional to the amount of free gentamycin present in the sample under uniform conditions (competitive assay approach). This solution is pumped through the flow cell yielding a current peak, the magnitude of which is directly proportional to the amount of hydrogen peroxide present. Since no enzyme label is present in the final solution, no further hydrogen peroxide generation occurs after removal of solution from the microtiter well, thus current peaks, as opposed to current steps are observed on liquid passage through the flow cell.

Microtiter plates were coated in carbonate/bicarbonate buffer pH 9.6 for 1 hour at 37°C, then washed and blocked with 0.5% w/v gelatin in PBS buffer pH 7.4. Plates were either used directly or stored at 4°C until required.

### Experimental protocol and results

Primary incubations with gentamycin-GOD, with or without free gentamycin, and in milk or buffer (PBS pH 7.4) were performed for 1 hour at 37°C. Gentamycin-GOD concentrations were varied between 1:1000 and 1:20 000. All incubations solutions had a total volume of 100 µl. Plates were then washed in PBS/Tween 20.

Substrate solution (200 µl of 0.1M Glucose in PBS pH 7.4/0.1M KCl) was then added.

After a given time period for substrate conversion, 150 µl of solution was pumped through the flow cell using a peristaltic pump set at a constant flow rate of 300 µl/min. Responses were measured as peak heights in nA or mA. Sensor cards were preconditioned for 5 seconds at 1V prior to operation at +350 mV (versus Ag/AgCl reference/counter electrode). Results are shown below in Table 3.

**Table 3a**

| | Antigentamycin | | Gentamycin-GOD dilution | | | Milk | Buffer | Inhibition | Substrate time (min) | Subst rate temp. (°C) |
|---|---|---|---|---|---|---|---|---|---|---|
| | 1:100 | 1:1000 | 1:1000 | 10000 | 1:20000 | | | | | |
| 1 | | x | | x | | | | 0-200 ppb | 5 | 60 |
| 2 | | x | | | x | | | 0-200 ppb | 5 | 60 |

Table 3a: Experimental parameters used in end-point measurement studies.

**Table 3b**

| Exp. | 200 ppb | 100 ppb | 50 ppb | 25 ppb | 10 ppb | 5 ppb | 0 ppb | blank |
|---|---|---|---|---|---|---|---|---|
| 1 | 6.6 (0) | 5.0 (0) | 7.5 (11) | 6.6 (25) | 9.0 (27) | 5.5 (18) | 100 (25) | 16.9 (50) |
| 2 | 25.0 (3.7) | 28.5 (6.7) | 28.5 (6.7) | 15.2 (12.5) | 10.5 (9) | 12.4 (7.6) | 100 (14.5) | 19.5 (33) |

Table 3b: Results from end-point measurement experiments nos. 3 & 4. Results are recorded as a percentage of positive signal (0 ppb = no inhibition = 100% signal). Bracketed data records CV (%), n is 2-4.

Considerable inhibition of the positive signal was seen for gentamycin concentrations as low as 5 ppb when using a 1:10 000 dilution of gentamycin-GOD conjugate. As expected when using such a low conjugate dilution, inhibited signals were indistinguishable from the background noise generated in the system.

### Embodiments of an Apparatus according to the Invention

Embodiments of will be further explained and illustrated, by way of example, with reference to the accompanying drawings, in which:
- Figure 1: is an elevational view of a flow cell, the top part partly broken away;
- Figure 2: is a cross-section of a flow cell along the line II - II of fig. 1;
- Figure 3: is a cross-section of a flow cell along the line III - III of fig. 1;
- Figure 4: is a schematic view of an apparatus containing a flow cell according to the invention, in a first embodiment;
- Figure 5: is a schematic view of an apparatus containing a flow cell according to the invention, in a second embodiment; and
- Figure 6: is a schematic view of a third embodiment of an apparatus according to the invention.

In the drawing corresponding elements have corresponding reference numbers.

In figs. 1 - 3, a flow cell 1 is shown, specifically for use in a method according to the present invention. The flow cell 1 comprises a first part 2, further referred to as bottom part 2 and a second part 3, further referred to as top part 3. The flow cells are all shown schematically and are not to scale. Proportions and dimensions can vary. Between the bottom part 2 and top part 3, a channel or similar vessel 4 is enclosed which connects a liquid inlet 5, on one side of the flow cell 1, with a liquid outlet 6 on the opposite side of the flow cell 1. The top part 3 is connected to the bottom part 2 by a hinge 7 along a side of the flow cell parallel to the length of the channel or similar vessel 4. Between the bottom part 2 and the top part 3 a seal 8 is positioned to prevent liquid passing between the parts 2 and 3 during use, other then through the channel or similar vessel 4.

On the bottom, 9, of the flow channel or similar vessel 4, a first electrode 10, to be referred to as working electrode (WE) is positioned near the liquid inlet 5. At least one second electrode 11, a reference (RE) or counter electrode (CE) is positioned on the bottom, 9 of the channel or similar vessel, 4, downstream from the working electrode 10. The, or each second or further electrode can also be positioned upstream or in any other position, distant from the first electrode, such that interference of the electrodes is avoided. The electrodes are preferably of a screen-printed type and can be either positioned on the channel or similar vessel bottom 9 or, preferably, in a recess in the channel or similar vessel bottom 9, such that the upper surface of the electrodes 10, 11 are flush with the surface of the channel or similar vessel bottom 9, in order to minimise turbulence during use.

Over the working electrode 10, a solid phase 12 is positioned, for example in the form of a sheet of nitro-cellulose or any other suitable solid phase. The sheet of solid phase 12 has a width somewhat greater then the width of the channel or similar vessel 4 perpendicular to flow direction thereof. The sheet 12 is provided with opposite side parts 13 clamped between the bottom part 2 and top part 3 when the flow cell 1 is in its closed position (fig. 2). The sheet 12 is preferably mainly provided with reactive solid phase on the side directed to the working electrode 10, while the working electrode 10 is covered by the sheet 12. During use liquids can pass trough the channel or similar vessel over the sheet 12, between the sheet 12 and the working electrode 10 as well as between the sheet 12 and the channel or similar vessel roof 14 opposite the channel or similar vessel bottom 9, thereby reacting with the solid phase.

The top part 3 can be pivoted to an opened position, shown in broken lines in fig. 2. In this opened position the channel or similar vessel 4, especially the electrodes 10, 11 and the solid phase 12 are directly accessible. The sheet 12 of solid phase can be taken out of the relevant part 2, 3 and be exchanged for a new sheet 12 of solid phase, after which the top part 3 can be brought in the closed position against the bottom part 2 again, clamping the solid phase in position. In the opened position furthermore the channel or similar vessel 4 forming parts, the electrodes 10, 11 and the seals can be handled, if necessary. This flow cell 1 therefore has the advantage that it can be re-used after exchange of the solid phase, which can be easily achieved.

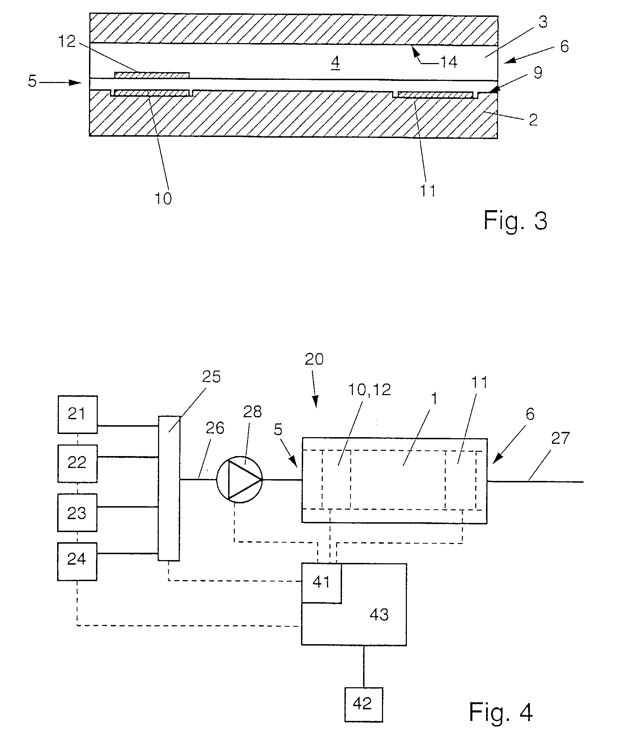

Fig. 4 shows a schematic view of an apparatus 20 containing a flow cell 1 according to the invention. The apparatus 20 comprises a first source 21 of a liquid suspected of comprising an analyte, for example milk, a second source 22 of a liquid comprising a substrate, a third source 23 of a liquid comprising a molecule having a specific binding affinity for the analyte and a fourth source 24 of a liquid containing a molecule having a label. The apparatus furthermore comprises a multi-valve 25, connected to the inlet-side with the said sources 21 - 24. The outlet-side of the multi-valve 25 is connected to a first conduit 26, connected to the liquid inlet 5 of the flow cell 1. A second conduit 27 is connected to the liquid outlet 6 thereof, for discharge of the liquids from the flow cell. In the first conduit 26 a pump 28 is positioned for pumping the liquids from the respective sources 21 - 24 to and through the channel or similar vessel 4. The pump 28 is preferably of a peristaltic type, capable of very precisely pumping small amounts of liquids, for example as little as 0.1 ml per charge. This enables very accurate dosage of the liquids.

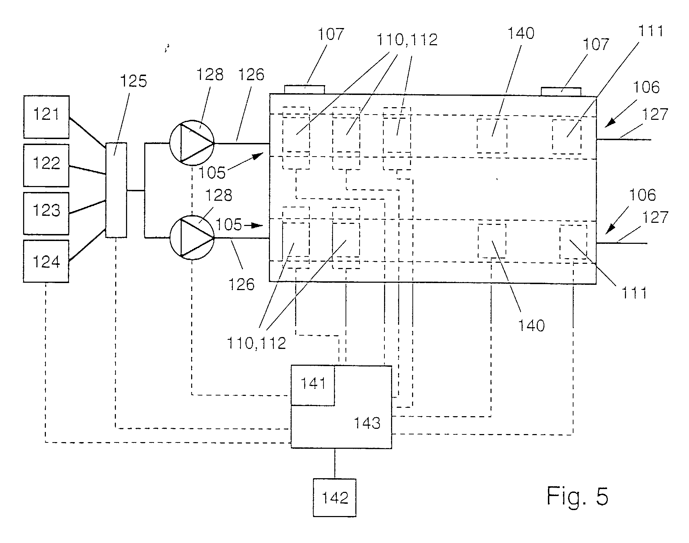

In fig. 5 a second embodiment of an apparatus according to the invention is shown schematically, wherein a flow cell 101 is provided with two parallel channels or similar vessels 104, each channel or similar vessel 104 provided with a series of measuring electrodes 110 and corresponding solid phases 112. Each solid phase 112 is intended for detecting a specific analyte. In this embodiment a mixing apparatus 130 is connected to at least two sources, in the embodiment shown as an example the first 121 and second source 122. In the mixing apparatus 130 a combination can be obtained of a specific dosage of the liquid from the connected sources, for example for pre-labeling of the analyte in the sample. The outlet-side of the mixing apparatus 130 is connected to an inlet-side of the multi-valve 125, as are the other sources 123 and 124. The outlet side of the multi-valve 125 is connected to the liquid inlets 105 of the respective channels or similar vessels 104 of the flow cell 101. It will be apparent that the channels or similar vessels 104 can also be provided in different flow cells, whereas any number of measuring electrodes can be positioned in any one of the channels or similar vessels.

In the channels or similar vessels 104 of the flow cell 101, a counter electrode 140 is positioned between the working electrode 110, at least downstream of the last working electrode 110, and the reference electrode 111 for enhancing the results of measurements, especially when high currents and/or high fluctuations in currents require detection.

The electrodes 10, 11 or 110, 111, 140 are connected to a measuring device 41, 141 respectively. Only the measuring device 41 will be described more extensively, the measuring device 141 being comparable to this device 41. The measuring device is provided with means for maintaining an operating potential across the electrodes 10, 11.

Furthermore the device 41 is provided with means for detecting current fluctuations, resulting from electrochemical reactions on or near the working electrode 10. The resulting fluctuations are presented by a means of a display 42 connected to the measuring device as, for example, absolute levels of measured current (mA) at a given reference time after initiation of the electrochemical reaction or as changes in the measured current in (δmA/sec).

Instead of one pump 28, 128 in the first conduit 26, 126 a pump can be positioned in the second conduit 27 or a number of pumps can be positioned in conduits between the respective sources 21 - 24, 121 - 124 and the multi-valve 25, 125.

The measuring device can be integrated in a control device 143, further comprising means for operating the sources 21 - 24, 121 - 124, the or each pump 28, 128, the multi-valve 25, 125 and/or the voltage over the electrodes 10, 11, 110, 111, 140. This control device 143 is preferably programmable and designed for (semi)automatic operation of the apparatus.

The measuring and/or control device 42, 142, 43, 143 is further provided with means for maintaining a potential difference between at least two of the electrodes, which difference can be positive or negative, relative to the or each working electrode, depending upon the particular reaction. Preferably, the means for obtaining said potential difference is designed for maintaining this difference at a relatively constant level, in order to obtain smooth signal data, which is readily available and interpretable and has a high signal/noise ratio.

Heating and/or cooling systems can be provided for regulating the temperature of the liquids.

In an alternative embodiment, as shown in fig. 6, a number of said sources 21-24, 121-124 are connected to the second conduit 27, 127, by means of a pump 28, 28', 128, 128' provided for directing liquids from said sources in either direction through the channel or similar vessel, depending upon the positions of the respective sources. In this embodiment a liquid can be expelled from the channel or similar vessel 4, 104 by feeding the next liquid to said channel or similar vessel, wherein the liquid is forced forward or backward through the channel or similar vessel wherein the liquid can be forced back to its initial source or can be expelled.

In the embodiments shown, the electrodes are positioned in the channel or similar vessel, at a fixed distance apart from each other. In a further preferred embodiment, not shown, the electrodes to be used in a flow cell 1, 101, a number of these electrodes, preferably all electrodes (WE, CE and/or RE) are positioned on a card or the like, preferably printed thereon by for example thick film screen-printing methodology, the card being easily replaceable. Contact pads or the like are provided within the flow cell for connection of the electrodes to the measuring device.

Part or all of the solid phase can be positioned directly on the working electrode thus providing for easy positioning and removal thereof and for protection of the said electrode, if necessary, against corrosive or otherwise reactive components within the liquids to be fed through the channel or similar vessel.

The invention is in no way limited to the embodiments as shown in the description and the drawings. Many modifications and variants are possible within the scope of the invention as defined in the enclosed claims. For example a number of flow cells or a number of channels or similar vessels can be connected to different sources of at least the first kind, that is sample sources, each cell or channel being connectable to the same second, third and/or fourth or further sources, depending on the particular operation undertaken. On the other hand one cell or channel or similar vessel can be connectable to a number of said sample sources, whereby means are provided for subsequently performing a full measuring cycle on a sample of each first source. These embodiments are for example useful when a number of similar samples require testing for similar analytes. Furthermore, the or each channel or similar vessel can have a different cross-section and flow path. The electrodes can have various forms and can be made of different materials or combinations thereof, mainly depending on the currents to be measured, the space available and the measurement conditions such as the solid phase to be used and the pH of the various liquids used. Different numbers of sources can be connectable to the or each flow cell. Furthermore the flow cell can be constructed in a different form, for example of two or more parts being fully separable from each other. The solid phase and/or the electrodes can be mountable within the channel or similar vessel in different ways, for example integrated onto one carrier which could be maneuvered into place to form a channel or similar vessel. A flow cell having such a construction would not require disassembly and re-assembly of the flow cell in order for replacement of the sensor device. Furthermore the solid phase could be positioned within the channel or similar vessel by other mounting means, for example along one side on the channel or similar vessel base, on the upstream side. A number of suitably prepared flow cells can be positioned in line and mounted in such a way that they can be adjusted to allow subsequent positioning of the flow cells subsequently between the first and second conduit to allow a series of measurements to be performed.

These and many similar variants are to be considered as falling within the scope of the invention.

## Claims

1. A method for determining the presence and/or the amount of at least one analyte in a liquid sample, comprising providing a molecule having specific binding affinity for said analyte, providing a label capable of generating or consuming an electrochemical signal detectable by an electrode, whereby the liquid sample flows along the electrode and whereby a solid phase is brought in close vicinity with said electrode, which solid phase is capable of binding a molecule having the label, whereby the presence of said label is detected by said electrode allowing determination of the presence and/or the quantity of analyte in the sample.

2. A method according to claim 1 whereby the electrode is a printed electrode, preferably a screen-printed electrode or wherein the electrode is prepared by any method that involves the gluing or otherwise fixing of the electrode into position.

3. A method according to claim 1 or 2 whereby the solid phase is a sheet of nitro-cellulose or the electrode itself.

4. A method according to claim 1, 2 or 3 whereby the label comprises an enzyme, preferably an oxidase, a reductase, a peroxidase, a redox enzyme or another enzyme resulting in a surplus or shortage of electrons, more preferably a glucoseoxidase or an alkaline phosphatase.

5. A method according to any one of claims 1-4 whereby the electrochemical signal is an electrochemically active species which is detectable by the electrode, preferably the electrochemically active species is hydrogen peroxide.

6. A method according to any one of claims 1-5 whereby a substantial proportion of the liquid sample is brought into intimate contact with the electrode.

7. A method according to any one of the aforegoing claims whereby a molecule having specific binding affinity for said analyte is an antibody, an antibody fragment, a receptor, a nucleic acid fragment, or a ligand for the analyte.

8. A method according to any one of the aforegoing claims whereby a specified amount of labeled analyte is provided.

9. A method according to anyone of claims 1-7 whereby a labeled molecule with specific binding affinity for the analyte is provided.

10. A method according to anyone of the aforegoing claims whereby the solid phase comprises a molecule having specific binding affinity for the molecule having the label preferably a labeled analyte.

11. A method according to anyone of the aforegoing claims, whereby the label is an enzyme and whereby a liquid sample flows along at least one electrode, comes into contact with a molecule having specific binding affinity for said analyte for a sufficient amount of time to allow binding to occur between analyte and specific binding molecule for said analyte, separating labeled material from unlabeled material using a solid phase, contacting said solid phase with a liquid comprising a substrate for said enzyme and measuring the surplus or shortage of electrons.

12. A method according to any one of the aforegoing claims whereby the liquid sample is an emulsion, a suspension or a colloidal solution.

13. A method according to anyone of the aforegoing claims whereby the liquid sample is a body fluid or a liquid derived therefrom.

14. A method according to anyone of the aforegoing claims whereby the liquid sample is milk or another dairy product.

15. A method according to anyone of the aforegoing claims whereby the liquid sample is derived from a food or feed product.

16. An apparatus for carrying out a method according to anyone of the aforegoing claims comprising a flow cell having a means, preferably a channel, through which liquid can flow whereby said means is provided with a working electrode, and at least one of a reference electrode and a counter electrode, a solid phase comprising a specific binding molecule capable of binding the molecule having the label, in close vicinity to said working electrode, said means having an inlet capable of being connected with one or more sources selected from the group consisting of a first source of a sample suspected of comprising an analyte, a second source of a liquid comprising a substrate, a third source of a liquid comprising a molecule having specific binding affinity for the analyte and a fourth source of a molecule having a label, whereby one or more of these sources may be one and the same, said means is provided with a liquid outlet, said electrodes suitable of being connected to a measuring device and the liquid in and/or outlet suitable of being connected to a liquid flow regulating means to flow the liquid.

17. An apparatus according to claim 16 whereby at least one electrode is a printed electrode, preferably a screen-printed electrode or the electrode is prepared by any method that involves the gluing or otherwise fixing of the electrode into position.

18. An apparatus according to claim 16 or 17 whereby the solid phase is a suitable porous polymeric material, preferably a sheet of nitro-cellulose.

19. An apparatus according to claim 16 or 17 whereby the electrode is at least partly made of a carbon material, preferably a catalytic carbon material, more preferably of a metallised carbon material which advantageously is a rhodinised carbon material.

20. An apparatus according to any one of claims 16 - 19, wherein any one of the sources comprises a combination of at least two of the sources.

21. An apparatus according to any one of claims 16 - 20, wherein the means is formed in a housing, the housing comprising a first part and a second part, the means being defined by the space between the first and a releasably connectable second part, the means being accessible between the liquid inlet and liquid outlet when the first part is at least partly separated from the second part, such that the electrodes and the solid phase are directly accessible from the outside of the means.

22. An apparatus according to claim 21, wherein the solid phase is provided with at least one side part, preferably two opposite side parts being positioned between the first and second part during use of the flow cell, thus positioning the solid phase relative to the means, at least partly within the flow path of liquids passing through the means, the solid phase being interchangeable for an other solid phase.

23. An apparatus according to any one of claims 16 - 22, wherein the working electrode is positioned near the liquid inlet, the or each other electrode being positioned downstream from the working electrode.

24. An apparatus according to any one of claims 16 - 23, wherein the solid phase is at least partly positioned over the working electrode.

25. An apparatus according to any one of claims 16 - 24, wherein the means has an at least mainly perpendicular or oval cross section, the solid phase being relatively flat and positioned at the base of the means, leaving a passageway between the free surface of the solid phase and the roof of the means opposite the base of the means and between the solid phase and the working electrode.

26. An apparatus according to any one of claims 16 - 25, wherein:
the first, second, third and/or fourth source are connected to respective inlets of a multi-valve means, a supply channel being connected between the outlet of the multi-valve means and the liquid inlet of the means, the contact of the dosing and liquid flow regulating means, the apparatus being provided for control of the multi-valve means and the liquid flow regulatory means, such that predetermined dosages can be directed from the sources through the means, depending on the sample to be analysed or assay format.

27. An apparatus according to claim 26, wherein mixing means are provided, connected to at least two or more sources and designed for mixing liquids form said connected sources, prior to supplying the mixture to the liquid inlet.

28. An apparatus according to any one of claims 16 - 27, wherein the liquid flow regulatory means comprises at least one dosage pump, preferably of a peristaltic type for dosage of small quantities of liquids.

29. An apparatus according to any one of the claims 16 - 28, wherein the flow cell comprises at least two working electrodes, each provided with a solid phase in the vicinity thereof.

30. An apparatus according to any one of the claims 16 - 29, wherein the flow cell comprises at least two means, each channel provided with at least one working electrode with a solid phase positioned in the vicinity thereof.

31. A flow cell for use in a method according to anyone of claims 1-15 or an apparatus according to anyone of claims 16-30.
